# EUROPEAN PATENT SPECIFICATION

(11) **EP 3 046 592 B1**
(45) Date of publication and mention of the grant of the patent: **05.09.2018**
(21) Application number: 14846600.6
(22) Date of filing: 18.09.2014
(51) Int. Cl.: A61L 15/46, A01N 37/06, A01N 65/08, A01P 1/00, A61F 13/496, A61L 15/22, A61L 15/34

(54) **HIGHLY ABSORBENT RESISTANT WASHABLE AND REUSABLE UNDERGARMENTS, METHODS OF USE, KITS AND USES THERETO**
IN HOHEM MASSE SAUGFÄHIGE UND ABWASCHBARE EINWEG-UNTERWÄSCHE, VERFAHREN ZUR VERWENDUNG, KITS UND VERWENDUNGEN DAFÜR
SOUS-VÊTEMENTS LAVABLES ET RÉUTILISABLES, TRÈS ABSORBANTS ET RÉSISTANTS, PROCÉDÉS D'UTILISATION, KITS ET UTILISATIONS ASSOCIÉS

(30) Priority: 19.09.2013 US 201361879913 P
(43) Date of publication of application: 27.07.2016
(73) Proprietor: Les Produits DRC Inc., Pierrefonds, Québec H8Y 0A1 (CA)
(72) Inventor: TULK, Raquel, Laval, Québec H7W 0G3 (CA); O'SHEA, Chanelle, Laval, Québec H7Y 2C4 (CA)
(74) Representative: Regimbeau
(86) International application number: PCT/CA2014/000702
(87) International publication number: WO 2015/039218

(56) References cited:
- WO-A1-01/41688
- WO-A1-2008/008743
- WO-A1-2011/075809
- WO-A2-92/03999
- CA-A1- 2 334 673
- CA-A1- 2 793 089
- CA-A1- 2 812 772
- CA-A1- 2 827 795
- GB-A- 2 176 692
- GB-A- 2 477 571

## Description

### FIELD OF THE DISCLOSURE

The present subject-matter relates to undergarments, and more particularly to undergarments that are highly absorbent, resistant, washable, and reusable.

### INTRODUCTION

Urinary incontinence is a condition affecting both men and women, which causes involuntary leakage of urine. According to some researches, 1 in 3 women are affected by this condition. This condition can often cause embarrassment, discomfort and even depression. Some causes of urinary incontinence are pregnancy, childbirth, menopause, exercise, post-surgery, and aging. In some cases, the actions of sneezing, coughing, or laughing can cause urinary leaks in some women who have a weakness in the bladder. For other women, the condition can be more severe. For example, some women feeling an urge cannot reach a washroom on time.

Excessive perspiration, feminine discharge, and menstruation are other causes that can lead to liquid being produced out of the vagina.

Disposable adult diapers, such as the ones described in WO 01/41688, provide a solution for urinary incontinence of both men and women. However, they can be uncomfortable and cause embarrassment to the wearer. This may result in psychological and social anxiety. Moreover, since they must be disposed, usage of many adult diapers can become expensive. Usage of many adult diapers may also be harmful to the environment due to the amount of materials needed. Washable and reusable undergarments are more advantageous. Such undergarments have notably been disclosed in WO 2011/075809, GB 2477571, GB 2176692, WO 2008/008743 and WO 92/03999.

### SUMMARY OF THE DISCLOSURE

It would thus be highly desirable to be provided with an article that would at least partially address the disadvantages of existing technologies.

According to one aspect, there is provided an undergarment comprising:
a fabric layer for wearing about a pelvic region of a person;
a water-repellent layer coupled to the fabric layer, the water-repellent layer and a crotch portion of the fabric layer defining an inner chamber, the water-repellent layer comprising polypropylene and being disposed for contacting a genital area of a person wearing the undergarment; and
an inner pad disposed within the inner chamber between the water-repellent layer and the fabric layer, the inner pad being reusable and comprising a liquid-absorbent layer and a waterproof layer, wherein the liquid-absorbent layer comprises tangled cellulose fibers chosen from bamboo, cotton, viscose and poly-micro; and the waterproof layer comprises polyurethane,
and the inner pad maintaining its shape and liquid-absorbent properties after being submitted to heat,
the liquid-absorbent layer contacting the water-repellent layer and being effective for absorbing a volume of liquid of about 88.7 mL (3 fluid ounces) to about 177.4 mL (6 fluid ounces),
the waterproof layer being effective for restricting liquid from reaching the fabric layer and the water-repellent layer being effective for repelling the liquid and urging the liquid to contact the liquid-absorbent layer;
and whereby the undergarment is reusable, renewable and resistant to heat.

According to another aspect, there is provided a method for reducing discomfort and/or embarrassment caused by incontinency,
the method comprises contacting an undergarment as defined in the present disclosure with a C8-C16 unsaturated fatty acid and/or an essential oil, and wearing the undergarment.

According to another aspect, there is provided a kit for reducing discomfort and/or embarrassment caused by incontinency, the kit comprising an undergarment as defined in the present disclosure and an antimicrobial agent, wherein the antimicrobial agent comprises a C8-C16 unsaturated fatty acid and/or an essential oil.

It was observed that by using the undergarments of the present disclosure, it was possible to provide to customers comfortable reusable undergarments that absorb high volumes of body liquids as opposed to previously proposed solutions. It was also observed that such undergarments were efficient for reducing the amount of material necessary for treating incontinency since these undergarments of the present disclosure are reusable. It was also found that the undergarments of the present disclosure are washable and resistant for example to heat and washing cycles. The undergarments of the present disclosure are thus efficient in helping the environmental cause.

### DRAWINGS

The following drawings represent non-limitative examples, in which:
Figure 1 illustrates a perspective view of an undergarment article according to various exemplary embodiments;
Figure 2 illustrates a top view of the undergarment article according to various exemplary embodiments;
Figure 2A illustrates an enlarged view of a crotch portion of the undergarment article according to various exemplary embodiments;
Figure 3 illustrates cross-sectional view along the lines A-A of a crotch portion of the undergarment article according to various exemplary embodiments;
Figure 4 illustrates an exploded view of the water-repellent layer, liquid-absorbent layer, waterproof layer and the fabric layer of the undergarment article according to various exemplary embodiments;
Figure 5 illustrates a cross-sectional view along the lines A-A of a crotch portion of the undergarment article according to an alternative exemplary embodiment;
Figure 6 illustrates a cross-sectional view along the lines A-A of a crotch portion of the undergarment article according to a further alternative exemplary embodiment; and
Figure 7 illustrates a top view of the undergarment article according to various alternative exemplary embodiments.

### DESCRIPTION OF VARIOUS EMBODIMENTS

The following examples are presented in a non-limiting manner.

For example, the undergarments of the present disclosure can be women undergarments or men undergarments.

For example, the undergarments of the present disclosure can be panties, bikini underwear, thongs, G-strings, swimsuits, nightwear, etc.

For example, the undergarments of the present disclosure can be shorts, boxer shorts, boxer briefs, swimsuits, nightwear, etc.

For example, the liquid-absorbent layer can be effective to absorb a volume of about 3 fluid ounces (88.7 mL) to about 6 fluid ounces (177.4 mL).

For example, the liquid-absorbent layer can be effective to absorb a volume of about 3 fluid ounces (88.7 mL) to about 5 fluid ounces (149.7 mL).

For example, the liquid-absorbent layer can be effective to absorb a volume of about 3 fluid ounces (88.7 mL) to about 4 fluid ounces (118.3 mL).

For example, the water-repellent layer can be liquid-permeable, wherein liquid contacting an exterior surface of the water-repellent layer can permeate through the water-repellent layer and can reach an interior surface of the water-repellent layer contacting the liquid-absorbent layer, the liquid-absorbent layer absorbing the liquid.

For example, the inner pad can be cleanable.

For example, the inner pad can maintain its shape and liquid-absorbent properties after being submitted to heat.

For example, the inner pad can be resistant to heat.

For example, the heat can correspond to a heat emitted from a clothes drying machine.

For example, the inner pad can be resistant to exposure from a heat from a flame positioned at about 1 to about 3 cm from the inner pad up to a duration of about 1 to about 3 seconds.

For example, the inner pad can be resistant to exposure from a heat from a flame positioned at about 1 to about 4 cm from the inner pad up to a duration of about 1 to about 4 seconds.

For example, the inner pad can be resistant to exposure from a heat from a flame positioned at about 1 to about 2 cm from the inner pad up to a duration of about 2 to about 3 seconds.

For example, the inner pad can be reusable, and whereby the undergarment is reusable, renewable and resistant to heat.

For example, the water-repellent layer can comprise a hydrophobic material.

For example, the water-repellent layer can comprise polypropylene.

For example, the water-repellent layer can comprise polypropylene and polyester.

For example, the water-repellent layer can comprise polypropylene and knitted polyester.

For example, the liquid-absorbent layer can comprise tangled cellulose fibers.

For example, the fibers can be chosen from bamboo, cotton, viscose and poly-micro.

For example, the waterproof layer can comprise polyurethane.

For example, the water-repellent layer can be coupled to at least one of a right lateral side and a left lateral side of a crotch portion of the fabric layer, the water-repellent layer and the crotch portion of the fabric layer can be defining an inner chamber, the crotch portion of the fabric layer and the water-repellent layer can be substantially elastic in at least one of a lateral direction and a longitudinal direction of the crotch portion.

For example, the inner pad can be disposed within the inner chamber between the water-repellent layer and the fabric layer. The inner pad can have a liquid-absorbent layer and a waterproof layer, the liquid-absorbent layer can be contacting the water-repellent layer, the waterproof layer can be effective for restricting liquid from reaching the fabric layer.

For example, the undergarments of the present disclosure can further comprise:
a first elastic trim coupled to the right lateral side of the crotch portion of the fabric layer and the water-repellent layer to form a first elastic seam; and
a second elastic trim coupled to the left lateral side of the crotch portion of the fabric layer and the water-repellent layer to form a second elastic seam.

For example, the first elastic trim can extend around the right leg opening and the second elastic trim extends around the left leg opening.

For example, the inner pad can be detached from the water-repellent layer and the fabric layer.

For example, the undergarments of the present disclosure can further comprise at least one stitch joining the water-repellent layer, the inner pad and the fabric layer at a location remote of the right lateral side and left lateral side of the crotch portion of the fabric layer.

For example, the undergarments of the present disclosure can further comprise at least one transverse stitch joins at least one end of the water-repellent layer with the fabric layer, the inner pad being detached from the transverse stitch; and
wherein the water-repellent layer and the fabric layer are elastic in a longitudinal direction of the crotch portion.

For example, at least a first transverse stitch joins a first end of the water-repellent layer with the fabric layer and at least a second transverse stitch can join a second end of the water-repellent layer with the fabric layer, the inner pad being detached from the at least first transverse stitch and the at least second transverse stitch; and
wherein the water-repellent layer and the fabric layer are elastic in a longitudinal direction of the crotch portion.

For example, a continuous stitch extending about the perimeter of the water-repellent layer can couple the water-repellent layer to the crotch portion of the fabric layer, the inner pad being detached from at least one transverse portion of the continuous stitch; and
wherein the water-repellent layer and the fabric layer are elastic in a longitudinal direction of the crotch portion.

For example, a continuous stitch extending about the perimeter of the water-repellent layer can couple the water-repellent layer to the crotch portion of the fabric layer, a first end of the inner pad being detached from at least a first transverse portion of the continuous stitch and second end of the inner pad being detached from at least a second transverse portion of the continuous stitch; and
wherein the water-repellent layer and the fabric layer are elastic in a longitudinal direction of the crotch portion.

For example, the water-repellent layer can be liquid-permeable, wherein liquid contacting an exterior surface of the water-repellent layer permeates through the water-repellent layer and reaches an interior surface of the water-repellent layer contacting the liquid-absorbent layer, the liquid-absorbent layer absorbing the liquid.

For example, the antimicrobial agent can comprise a C8-C16 unsaturated fatty acid and/or an essential oil.

For example, the antimicrobial agent can be in an aqueous composition that comprises a C8-C16 unsaturated fatty acid and/or an essential oil.

For example, the antimicrobial agent can comprise a fatty acid.

For example, the antimicrobial agent can comprise a fatty acid that is undecylenic acid.

For example, the essential oil can be an essential oil chosen from spices and herbs (for example thyme, origanum, mint, cinnamon, salvia and clove).

For example, the antimicrobial agent can be chosen from organic acids (sorbic acid, propionic acid, acetic acid, lactic acid, citric acid, malic acid, benzoic acid) and their salts, PHB esters and their salts, sodium sulphite and corresponding salts, nisin, natamycin, formic acid, hexamethylenetetramine, sodium tetraborate, lysozyme, alcohols, organic halogen compounds, parabens (methyl, ethyl, propyl, butyl, isobutyl and propyl paraben), isothiazolones (benzisothiazolone, methylisothiazolone, octylisothiazolone), phenols, salicylates, nitriles, fragrances, flavours, and other active plant or synthetic ingredients with antimicrobial activity, and also metal salts, such as zinc oxide, zinc pyrithione and zinc alginate, for example.

For example, the antimicrobial agent can be, for example, menthol, peppermint oil, geraniol, limonene, citronellol, nerol, farnesol, tea tree oil, cinnamic acid, lemon grass oil, capsaicin, tannic acid, seaweed, sea grass and lemon grass (oil essences); grapefruit seed, garlic, allicin, peat (extracts); peppermint oil, sage oil, thymol, eugenol and *Thuja orientalis* (extracts);, *eucalyptus* oil and ginger; olive leaf extract; alpha-pinene and beta-pinene extracts; rhubarb root extract; tea tree oil (terpinen-4-ol, alpha-terpinene, gamma-terpinene, alpha-terpineol, terpinolene); thyme (extract) and vitamin E (extract).

For example, the fatty acid can be present in an aqueous composition.

For example, the fatty acid can be comprised within microcapsules.

For example, the microcapsules can comprise a copolymer of methyl vinyl ether and maleic anhydride.

For example, the antimicrobial agent can comprise an essential oil.

For example, the antimicrobial agent can be effective for treating undesirable odors.

For example, the antimicrobial agent can be effective for at least partially masking undesirable odors.

For example, the microencapsulated aqueous composition can comprise microcapsules comprising the aqueous composition comprising undecylenic acid encapsulated, wherein the microcapsules comprise a copolymer of methyl vinyl ether and maleic anhydride.

For example, the contacting can comprise impregnating the undergarment with the C8-C16 unsaturated fatty acid and/or the essential oil.

For example, the contacting can comprise contacting the water-repellent layer of the undergarment with the C8-C16 unsaturated fatty acid and/or the essential oil.

For example, the essential oil can be an essential oil chosen from spices and herbs (for example thyme, origanum, mint, cinnamon, salvia and clove).

For example, the undergarment can be treated with the antimicrobial agent. For example, it can be impregnated with the antimicrobial agent. For example, the antimicrobial agent can be applied on the fabric layer and/or the water-repellent layer. For example, the antimicrobial agent can be applied on the water-repellent layer.

For example, the antimicrobial agent can be undecylenic acid.

For example, the antimicrobial agent can be an aqueous composition comprising undecylenic acid.

For example, the antimicrobial agent can be a composition as follows :

| **Component** | **Weight %** |
|---|---|
| Water | 61-65 |
| Essential oil | 25-35 |
| PVM / MA Copolymer | 2-4 |
| Melamine / Formaldehyde Resin | 2-4 |
| Sodium Hydroxide | 0.5-1.5 |
| Oxalic Acid | 0.5-2 |
| Melamine | 0.1-0.5 |

For example, such composition can be under the form of microcapsules.

For example, the microcapsule can be prepared as follows :
- Emulsifying the oil at a concentration of about 30 wt % (based on the total weight of the composition) in an aqueous phase containing the other components;
- Polymerization under agitation at a controlled temperature in order to form the microcapsules (shell of polymer and a liquid core i.e. the aqueous composition (liquid core) is encapsulated by the shell of polymer) at the oil/water interface; and
- Cooling until room temperature is reached.

For example, the microcapsules can be prepared in a thermo-regulated vessel or reactor.

For example, the antimicrobial agent can be a composition as follows :

| **Component** | **Weight %** |
|---|---|
| Water | 60-70 |
| Paraffinum Liquidum (liquid paraffin) | 15-25 |
| Perfume | 2-6 |
| Undecylenic acid | 2-6 |
| PVM/MA Copolymer | 2-4 |
| Methoxypolyoxymet hylene melamine | 2-4 |
| Sodium Hydroxide | 0.5-1.5 |
| Oxalic Acid | 0.5-2 |
| Melamine | 0.1-0.4 |

For example, such composition can be under the form of microcapsules.

For example, the microcapsule can be prepared as previously described.

For example, the antimicrobial agent can be applied on the undergarment before wearing it.

For example, the antimicrobial agent can be applied on the undergarment after washing the undergarment.

For example, the antimicrobial agent can be applied on the undergarment after washing the undergarment, while the undergarment is still wet.

For example, the antimicrobial agent can be applied on the undergarment after washing and drying the undergarment.

For example, the antimicrobial agent can be applied on the undergarment after washing, but prior to drying the undergarment.

For example, the kit can comprise instructions for treating the undergarment with the antimicrobial agent.

For example, the kit can comprise instructions for impregnating the undergarment with the antimicrobial agent.

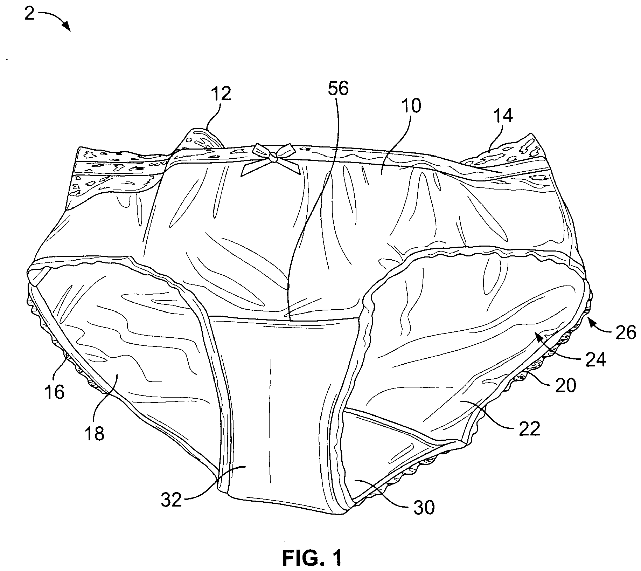

Referring now to Figure 1, therein illustrated is a perspective view of an undergarment article 2 according to various exemplary embodiments. The undergarment article 2 has a fabric layer 10. The fabric layer 10 is shaped so that it can be worn about a pelvic region of a person. A top side 12 of the fabric layer 10 defines a torso opening 14. A right side 16 of the fabric layer 10 defines a right leg opening 18 and a left side 20 of the fabric layer 10 defines a left opening 22. When the undergarment article 2 is worn by the person, the top side 12 of the fabric layer contours the person's torso, which passes through the torso opening 14, the right side 16 of the fabric layer 10 contours the right leg of the person, which projects through the right leg opening 18, and the left side 20 of the fabric layer 10 contours the left leg of the person, which projects through the left leg opening 22.

The fabric layer 10 can be seamless (formed of one piece of fabric) or can have one or more seams (formed of a plurality of pieces of fabric). Where the fabric layer 10 is formed a plurality of pieces of fabric, the pieces can be joined together according to known undergarment patterns.

The fabric layer 10 can have various shapes according known undergarment patterns. For example, the fabric layer 10 can be mid-waist, high-waist, low-waist, bikini cut, thong, boy cut, tummy control panties, classic brief, hipster, tanga, and G-string

The fabric layer 10 can be formed of fabric material typically used for undergarments, such as cotton, polyester, silk, lace, or a combination thereof. Where the fabric layer 10 is formed of a plurality of pieces of fabric, more than one fabric material may be used, such as a cotton core with lace trims.

The fabric layer 10 has an inner surface 24 and an outer surface 26. When worn by the person, portions of the inner surface 24 may contact the skin of the person.

The undergarment 2 further has a water-repellent layer 30, which is positioned on an inner surface 24 of the fabric layer 10. The water-repellent layer 30 covers at least the inner surface 24 of a crotch portion 32 of the fabric layer 10. The crotch portion 32 is a portion of the fabric layer 10 that is positioned over the crotch area, including the genital area, of the person when the undergarment article 2 is worn. For example, the water-repellent layer 30 has a generally hour-glass planar shape to substantially cover the crotch area of the person.

According to various exemplary embodiments, the water-repellent layer 30 comprises polypropylene.

According to various exemplary embodiments, the water-repellent layer 30 comprises polypropylene and polyester.

According to various exemplary embodiments, the water-repellent layer 30 comprises a mixture of polypropylene and knitted polyester. For example, the water-repellent layer 30 is a two-ply material having a first polypropylene ply and a second knitted polyester ply. For example, the water-repellent layer 30 is formed of about 40% polypropylene and about 60% polyester knitted fabric.

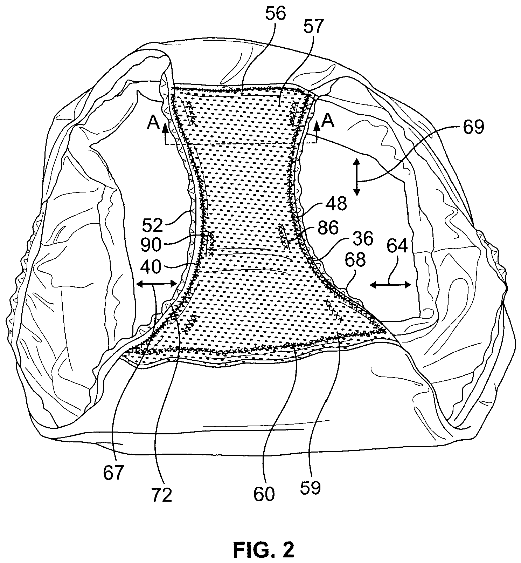

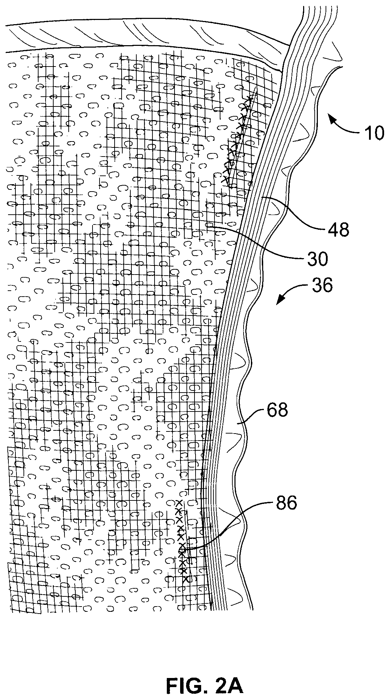

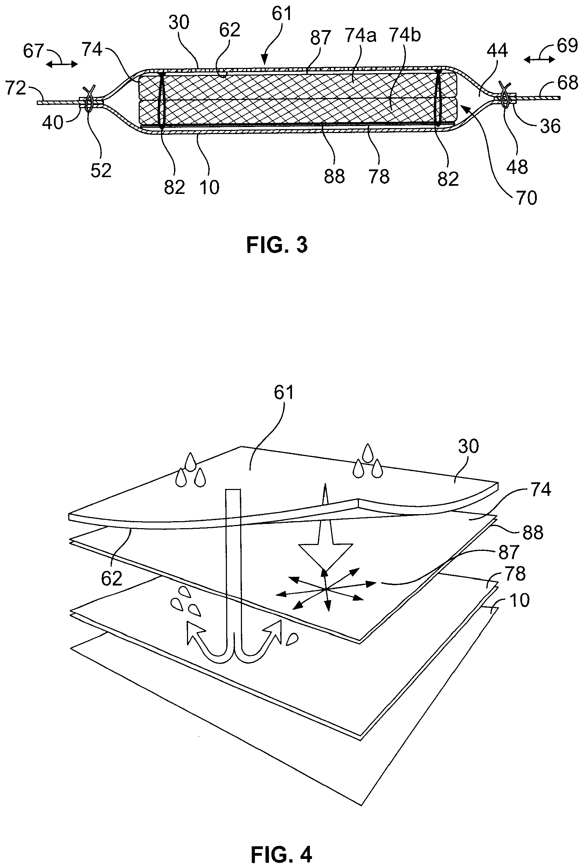

Referring now to Figure 2 and 2A, therein illustrated are a top view of the undergarment article 2 and a top view of a side of the crotch portion 32 of the undergarment article 2 respectively, according to various exemplary embodiments. It can be seen that the water-repellent layer 30 covers the crotch portion 32 of the fabric layer 10. According to various exemplary embodiments, the water-repellent layer 30 is coupled to at least a right lateral side 36 and a left lateral side 40 of the crotch portion 32 of the fabric layer 10 to define an inner chamber 44 (Fig. 3). For example, a first stitch 48 along the right lateral side 36 and a second stitch 52 along the left lateral side 40 join the water-repellent layer 30 to the crotch portion 32 of the fabric layer 10.

For example, a first transverse stitch 56 at a first end 57 of the water-repellent layer 30 can be added to better join the water-repellent layer 30. It will be appreciated that the first transverse stitch 56 is made at an end corresponding to the front side of the person when the undergarment article 2 is worn. Additionally or alternatively, a second transverse stitch 60 at a second end 59 of the water-repellent layer 30 may be further added to better join the water-repellent layer 30 and the fabric layer 10. It will be appreciated that the second transverse stitch 60 is made at an end corresponding to the backside of the person when the undergarment article 2 is worn. The addition of the first transverse stitch 56 and/or second transverse stitch 60 is made at the ends of the water-repellent layer 30 so that it still defines the inner chamber 44 with the crotch portion 32 of the fabric layer 10.

The water-repellent layer 30 forms an exterior layer of the undergarment article 2 in that the outer surface 61 (Fig. 3) of the water-repellent layer 30 contacts the skin of the person when the undergarment article 2 is worn. For example, a portion of the water-repellent layer 30 contacts the genital area of the person.

According to various exemplary embodiments, the water-repellent layer 30 is formed of a liquid-permeable material that allows a body liquid to pass between the outer surface 61 (FIG. 3) and an inner surface 62 (FIG. 3) of the water-repellent layer 30. In fact, the body liquid will be forced to pass through the layer 30 and to be absorbed by the liquid-absorbent layer 74. The water-repellent layer 30 allows for keeping dry the skin and/or hair of a user that contact the outer surface 61 since body liquids will be repelled from the outer surface 61 and urged to pass through the inner surface 62 so as to contact the liquid-absorbent layer 74 and to be absorbed by the layer 74. When worn by a person, liquid leaked from the person flows from an outer surface 61 of the water-repellent layer 30 towards an inner surface 62 of the water-repellent layer 30. For example, the flow of liquid can be aided by the force of gravity. Exterior surface of the water-repellent layer 30 herein refers to a surface contacting the person when the undergarment article is worn around the pelvic region. Interior surface of the water-repellent layer 30 herein refers to a surface not contacting the person when the undergarment article is worn. The inner surface 62 of the water-repellent layer 30 also defines a surface of the inner chamber 44. Accordingly, the outer layer 61 of the water-repellent layer 30 feels less wet to the wearer, thereby improving comfort. For example, excess liquid or humidity remaining on the outer surface 61 is allowed to evaporate (in view of the liquid-repellent property), thereby further contributing to a less wet feel.

According to various exemplary embodiments, fabric layer 10 and water-repellent layer 30 are both formed of elastic materials. For example, the water-repellent layer 30 is coupled to at least the right and left lateral sides 36, 40 of the crotch portion 32 of the fabric layer 10 such that the water-repellent layer 30 and the crotch portion 32 of the fabric layer 10 are elastic in at a lateral direction of the crotch portion between the two leg openings 18, 22. For example, the lateral direction between the two leg openings is defined by arrows 64 and 67. It will be appreciated that the lateral direction defined by the arrows 64 and 67 represent a side-to-side direction of the undergarment article 2, which is transverse to a front-to-back axis of the undergarment article 2. For example, the first stitch 48 and the second stitch 52 can include elastic material, such as lycra, to provide elasticity..

Additionally or alternatively, the water-repellent layer 30 is coupled to at least the right and left lateral sides 36, 40 of the crotch portion 32 of the fabric layer 10 such that the water-repellent layer 30 and the crotch portion 32 of the fabric layer 10 are elastic in at least a longitudinal direction of the crotch portion defined by arrow 69. It will be appreciated that the longitudinal direction represents a front-to-back axis of the undergarment article 2.

According to various exemplary embodiments, the water-repellent layer 30 and each of the lateral sides 36, 40 of the crotch portion 32 of the fabric layer 10 are further coupled to an elastic trim 68 extending along the sides of the crotch portion 32. As shown in Figure 2, a right elastic trim 68 is coupled, for example through the right stitch 48, to the water-repellent layer 30 and the right lateral side 36 to form a right elastic seam. A left elastic trim 72 is coupled, for example, through the left stitch 52, to the water-repellent layer 30 and the left lateral side 40 to form a left elastic seam. The elastic trims 68, 72 improve longitudinal elasticity of the water-repellent layer 30 and the crotch portion 32 of the fabric layer 10. The elastic trims 68, 72 may further improve lateral elasticity of the water-repellent layer 30 and the crotch portion 32 of the fabric layer 10. The elastics trims 68, 72 may be formed using additional fabric pieces, such as knit lace edging. Alternatively, the elastic trims 68, 72 may be formed by folding back edges of the fabric layer 10 so as to provide fabric strips having a defined width between the lateral sides 36, 40 and the stitches 48,52.

According to one exemplary embodiment, the right elastic trim 70 extends about the entire length of the right side 16 of the fabric layer 10 defining the right leg opening 18 and the left elastic trim 72 extends about the entire length of the left side 20 of the fabric layer 10 defining the left leg opening 22.

Advantageously, lateral elasticity of the water-repellent layer 30 and the crotch portion 32 of the fabric layer 10 allows the leg openings 18, 22 to better contour and conform to the legs and crotch of the person wearing the undergarment article 2. This increases comfort and/or fit of the undergarment article 2 when worn. According to embodiments where the elastic trims 70, 72 extend about the entire length of the leg openings 18, 22, comfort and fit can be further improved. The elastic trims 70, 72 may further aid in restricting liquid contacting the water-repellent layer 30 from flowing past the sides 36, 40 of the crotch portion 32.

Referring now to Figure 3, therein illustrated is a cross-section view of the crotch portion 32 of the undergarment article 2 along the lines A-A. As described above, the water-repellent layer 30 is coupled to the right lateral side 36 and left lateral side 40 of the crotch portion 32 of the fabric layer 10 to define an inner chamber 44. For example, the coupling can be provided by stitches 48 and 52. The lateral sides 36, 40 and the water-repellent layer 30 can be further joined to elastic trims 68 and 72 to form the right elastic seam and the left elastic seam.

Continuing with Figure 3, an inner pad 70 is disposed inside the inner chamber 44 between the water-repellent layer 30 and the fabric layer 10. The inner pad 70 has a liquid-absorbent layer 74 and a waterproof layer 78. The liquid absorbent layer 74 and the waterproof layer 78 are aligned and disposed against each other. For example, the liquid absorbent layer 74 and the waterproof layer 78 are coupled together by one or more stitches 82. For example, the liquid absorbent layer 74 and the waterproof 78 have substantially the same planar shape. For example, the inner pad 70 has a planar shape corresponding to the generally hour-glass planar shape of the water-repellent layer 30 and the crotch portion 32 of the fabric layer 10. For example, planar area of the water-repellent layer 30 is slightly larger than the planar area inner pad 70 so that the inner pad 70 fits snugly with the inner chamber 44.

The liquid-absorbent layer 74 is formed of a material that is highly absorbent to liquid and has good liquid retention. For example, the liquid-absorbent layer 74 is formed of tangled cellulose fibers. For example, the tangled cellulose fibers may be chosen from bamboo, cotton, viscose, poly-microfiber or a combination of one or more thereof. According to one exemplary embodiment, the liquid-absorbent layer 74 includes Zorb™ material manufactured by Wazoodle Inc. The amount of material used to form the liquid-absorbent layer 74 is selected so that the inner pad 70 can retain a volume of liquid greater than 2 fluid ounces (59.1 mL).

The amount of material forming the liquid-absorbent layer 74 is selected to retain a volume of liquid greater than 2 fluid ounces is selected specifically to address the problems associated with urinary incontinence. While absorption below 2 fluid ounces is sufficient for light leaks, such as excessive perspiration and feminine discharge, only a portion of the causes of leakage is addressed by this range of volumes. Many cases of urinary incontinence are associated with leakages having a volume greater than 2 fluid ounces. These cases are addressed by selecting an amount of material forming the liquid-absorbent layer 74 to retain a volume of liquid greater than 2 fluid ounces.

According to one exemplary embodiment, the amount of material forming the liquid-absorbent layer 74 is selected to be effective to absorb a volume of liquid of about 3 fluid ounces (88.7 mL) to about 6 fluid ounces (177.4 mL).

According to one exemplary embodiment, the amount of material forming the liquid-absorbent layer 74 is selected to be effective to absorb a volume of about 3 fluid ounces (88.7 mL) to about 5 fluid ounces (149.7 mL).

According to various exemplary embodiments, the liquid-absorbent layer 74 is formed of one or more sub-layers of the tangled cellulose fibrous material. The number of sub-layers used depends on the desired liquid-absorption properties. As shown in Figure 3, a first sublayer 74a and a second sublayer 74b are disposed one above another. It has been found that two sub-layers of the Zorb™ material provide a liquid-retention ability of the inner pad 70 of more than 2 fluid ounces and up to about 3 ounces. It has been further found that three sub-layers of Zorb™ material provide a liquid-retention ability of the inner pad 70 of up to about 5 fluid ounces. Each of the sub-layers and the waterproof layer 78 can be joined together by stitches 82.

According to various exemplary embodiments, the liquid-absorbent layer 74 is formed of a material that is highly resistant to heat. For example, the liquid-absorbent layer is formed of a material that can withstand heat up to the maximum heat emitted from a clothes drying machine. The terms "withstanding heat" or being "resistant to heat" used herein in relation to the liquid-absorbent layer 74 refers to the liquid-absorbent layer 74 maintaining its shape and liquid retention properties after being exposed to heat.

According to various exemplary embodiments, the liquid-absorbent layer 74 is further formed of a material that is washable. For example, the liquid-absorbent layer 74 is formed of a material that can be exposed to standard cleaning products used for washing clothes, such as cloth-washing detergent, soap or bleach. The terms "washable" or "cleanable" used herein in relation to the liquid-absorbent layer 74 refers to the liquid-absorbent layer 74 maintaining its shape and liquid retention properties after being exposed to standard cleaning products used for washing clothes. Moreover, substances contained in liquid absorbed by the liquid-absorbent layer 74, such as bacteria and odors from urinary leakage, can be washed away when the liquid-absorbent layer 74 is exposed to standard cleaning products.

The waterproof layer 78 is formed of a material that is highly impermeable to liquid. For example, the waterproof layer 78 includes polyurethane.

According to various exemplary embodiments, the waterproof layer 78 is formed of a material that is highly resistant to heat. For example, the waterproof layer 78 is formed of a material that can withstand heat up to the maximum heat emitted from a cloth drying machine. The terms "withstanding heat" or being "highly resistant to heat" used herein in relation to the waterproof layer 78 refers to the waterproof layer 78 maintaining its shape and liquid impermeable properties after being exposed to heat.

According to various exemplary embodiments, the waterproof layer 78 is further formed of a material that is cleanable. For example, the waterproof layer 78 is formed of a material that can be exposed to standard cleaning products used for washing clothes, such as cloth-washing detergent, soap, or bleach. The terms "washable" or "cleanable" used herein in relation to the waterproof layer 78 refers to the waterproof layer 78 maintaining its shape and impermeability after being exposed to standard cleaning products used for washing clothes.

As a result of the liquid-absorbent layer 74 being cleanable and heat-resistant and the waterproof layer 78 also being cleanable and heat-resistant, the inner pad 70 is also resistant to heat and cleanable. Consequently, inner pad 70 is reusable. Furthermore, the entire undergarment article 2 is washable, reusable, and resistant to heat. Advantageously, by being reusable, the undergarment article 2 is more environmentally friendly than disposable options treating urinary incontinence, such as adult diapers. As a result of being reusable, the undergarment article 2 also becomes more cost effective than disposable options.

Continuing with Figure 3, the inner pad 70 is disposed within the inner chamber 44 so that a surface of the liquid-absorbent layer 74 contacts the water-repellent layer 30. As shown in Figure 3, a top surface 87 of the liquid-absorbent layer 74 contacts an interior surface 62 of the water-repellent layer 30. The waterproof layer 78 is joined to a bottom surface 88 of the liquid-absorbent layer 74 that is opposite the top surface 87. For example, the waterproof layer 78 corresponds to a layer adjacent to the fabric layer 10. For example, the waterproof layer 78 may be in contact with the fabric layer 10.

Referring now to Figure 4, therein illustrated is an exploded view of the water-repellent layer 30, the liquid-absorbent layer 74, the waterproof layer 78 and the fabric layer 10. Liquid expelled from a person reaches the outer surface 61 of the water-repellent layer 30 in contact with the genital area of a person. Since the water-repellent layer 30 is liquid permeable, the expelled liquid permeates through the water-repellent layer 30 and reaches the inner surface 62 of the water-repellent layer 30. The expelled liquid then contacts the top surface 87 of the liquid-absorbent layer 74, which absorbs the expelled liquid. The expelled and absorbed liquid spreads throughout the liquid-absorbent layer 74. A large portion of the liquid is retained within the liquid-absorbent layer 74, but a small portion of the liquid may flow from the bottom surface 88 of the liquid-absorbent layer 74 to the waterproof layer 78. Since the waterproof layer 78 is impermeable to liquid, the expelled liquid is restricted from reaching the fabric layer 10, thereby ensuring that the liquid stays in the liquid-absorbent layer 74 and that the fabric layer 10 stays substantially dry. Accordingly, liquid expelled from the person does not reach other clothing articles worn by the person, thereby preventing the appearance of potentially embarrassing wet spots and ensuring comfort of the wearer.

Referring back to Figure 3, according to one exemplary embodiment, the inner pad 70 is disposed within the inner chamber 44 such that the inner pad 70 is detached from the water-repellent layer 30 and the crotch portion 32 of the fabric layer 10. For example, stitches 48, 52 only join together the lateral sides 36, 40 of the crotch portion 32 of the fabric layer 10 with the water-repellent layer 30 but do not further join the inner pad 70. Alternatively, the stitches 48, 52 further join only the elastic trims 68, 72 to the water-repellent layer 30 and fabric layer 10. Similarly, stitches 82 only joins the liquid-absorbent layer 74 and the waterproof layer 78 but does not further join the water-repellent layer 30 and/or the fabric layer 10. In many cases, either or both of the liquid-absorbent layer 74 and the waterproof layer 78 are formed of an inelastic material. Using the water-repellent layer 30 and the fabric layer 10 to define an inner chamber 44 in which to position the inner pad 70 ensures that the inner pad 70 is properly aligned with the water-repellent layer 30 and the crotch portion 32 of the fabric layer. For example, a snug fit of the inner pad 70 within the inner chamber 44 improves the maintaining of the alignment. Detachment of the water-repellent layer 30 and the fabric layer 10 from the inner pad 70 maintains the lateral elasticity of the water-repellent layer 30 and the fabric layer 10, thereby improving the fit and comfort of the undergarment article 2 when worn. It will be appreciated that the lateral elasticity is maintained despite the inelasticity of the inner pad 70.

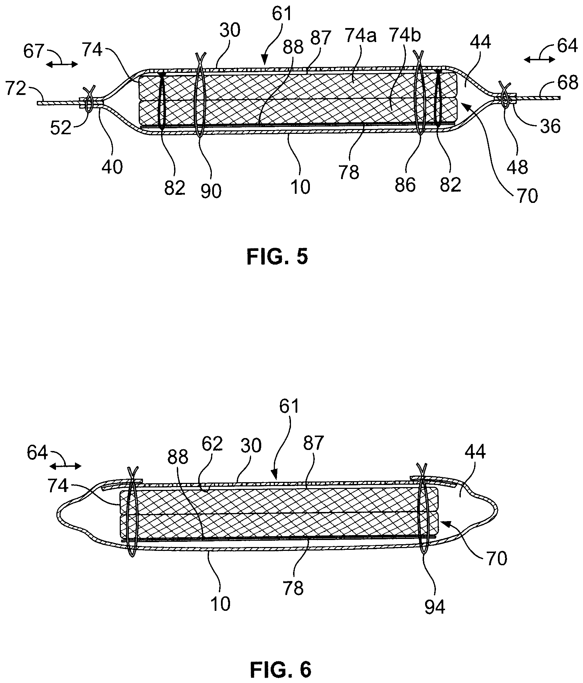

Referring now to Figure 5, therein illustrated is a cross-sectional view along the line A-A of the undergarment article 2 according to an alternative exemplary embodiment. According to the alternative exemplary embodiment, at least one stitch 86 further joins the water-repellent layer 30, the inner pad 70, and the fabric layer 10. As shown, the stitch 86 is made at a location remote from a lateral side of the crotch portion 32 of the fabric layer 10. For example, the stitch 86 is separated by a distance d from the right lateral side 36 of the crotch portion 32 of the fabric layer 10. For example, the stitch 82 of the inner pad is positioned between the location of the stitch 86 and right lateral side 36. The stitch 86 ensures proper attachment and alignment of the inner pad 70 with the water-repellent layer 30 and fabric layer 10. The separation between the stitch 86 and the lateral side 36 ensures that the water-repellent layer 30 and the fabric layer 10 remains substantially elastic in the lateral direction despite the attachment to the inelastic inner pad 70.

As shown a second stitch 90 is made at a location remote of the left lateral side 40 of the crotch portion 32 of the fabric layer 10.

According to an alternative exemplary embodiment, the at least one stitch extends in a direction parallel to a lateral side of the crotch portion 32 of the fabric layer 10. As shown in Figure 2, stitch 86 extends along a right lateral side 36 and stitch 90 extends along a left lateral side 40 of the crotch portion 32 of the fabric layer 10. More than one stitch remote of the lateral side may be formed. According to one exemplary embodiment, the stitches only extend along a portion of the side of the fabric layer so as to form discontinuities therebetween. The discontinuities further provide for elasticity of the water-repellent layer 30 and the fabric layer 10 in the lateral direction.

Referring now to Figure 6, therein illustrated is a cross-sectional view along the line A-A of the undergarment article 2 according to a further alternative exemplary embodiment. According to the alternative exemplary embodiment, at least one stitch 94 joins the water-repellent layer 30, the inner pad 70, and the fabric layer 10 at a lateral side of the crotch portion 32 of the fabric layer 10. The stitch 94 ensures proper attachment and alignment of the inner pad 70 with the water-repellent layer 30 and fabric layer 10. Accordingly, the first transverse stitch 56 and/or the second transverse stitch 60 only join the first and/or second ends 57, 59 of the water-repellent layer 30 and the fabric layer 10 while the inner pad 70 is detached from the first transverse stich 56 and the second transverse stitch 60. This detachment allows the water-repellent layer 30 and the fabric layer 10 to remain substantially elastic in the longitudinal (front-to-back) direction defined by arrow 69.

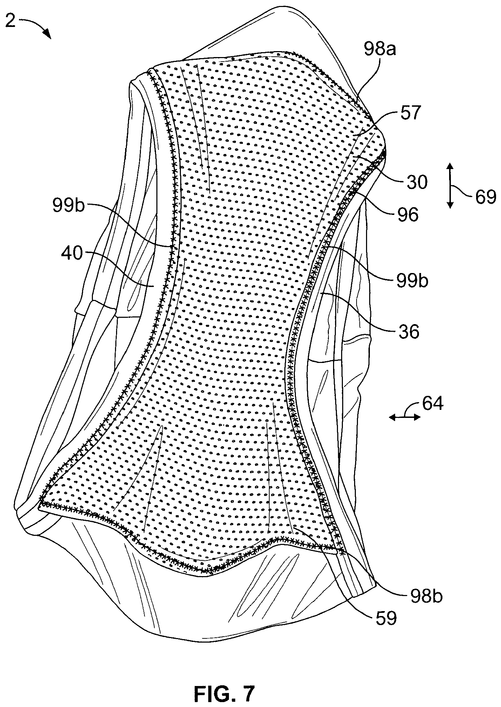

Referring now to Figure 7, therein illustrated is a top view of the undergarment article 2 according to various exemplary embodiments. According to various exemplary embodiments, the water-repellent layer 30 and the crotch portion 32 of the fabric layer 10 can be joined by one continuous stitch 96 extending about the perimeter of the water-repellent layer 30. For example, at least a transverse portion of the continuous stitch 96 joins at least one end of the water-repellent layer 30 with a corresponding end of the crotch portion of the fabric layer 10, but is detached from the inner pad 70. For example, first transverse portion 98a joins a first end 57 of the water-repellent layer 30 with the fabric layer 10 but is detached from a first end of the inner pad 70 and a second transverse portion 98b joins a second end 59 of the water-repellent layer 30 with the fabric layer 10 but is detached from a second end of the inner pad 70. Accordingly, the water-repellent layer 30 and the crotch portion of the fabric layer 10 are elastic in a longitudinal direction of the crotch portion 32 defined by arrow 69.

For example, at least a longitudinal portion of the continuous stitch 96 joins at least one lateral side of the water-repellent layer 30 with a corresponding side of the crotch portion of the fabric layer 10, but is detached from the inner pad 70. For example, first longitudinal portion 99a joins the water-repellent layer 30 to the right lateral side 36 of the crotch portion 32 and second longitudinal portion 99b joins the water-repellent layer 30 to the left lateral side 40 of the crotch portion 32. Accordingly, the water-repellent layer 30 and the crotch portion of the fabric layer 10 are elastic in a lateral direction of the crotch portion 32 defined by arrow 64.

For example, the continuous stitch 96 is detached completed from the inner pad 70. Accordingly, the water-repellent layer 30 and the crotch portion of the fabric layer 10 are elastic in both a lateral direction and longitudinal direction of the crotch portion. In some exemplary embodiments, a stitch remote from a lateral side 40, 42 of the water-repellent layer 30 can join the water-repellent layer 30, fabric layer 10, and inner pad 70 while maintaining lateral elasticity of the water-repellent layer 30 and crotch portion of the fabric layer 10.

### EXPERIMENT 1

According to a first experiment, the absorbency of the undergarment article 2 was compared with a commercially available liquid-absorbent underwear from Stride™. In particular, the first experiment tested absorbency for a volume of liquid greater than 2 fluid ounces, such a volume being associated with urinary incontinence.

A volume of colored liquid solution was prepared.

The undergarment article 2 was placed on a first sheet of a liquid-absorbent material with the crotch portion of the fabric layer contacting the sheet. The sheet can be a sheet of paper towel that will absorb and show the color liquid solution.

The Stride™ underwear was also placed on a second sheet of the liquid absorbent material with the crotch portion of the Stride™ underwear also contacting the sheet.

A volume of approximately 1 fluid ounce of the prepared liquid solution was excreted over an inner surface of the crotch portion of the Stride™ underwear. It was observed that the colored liquid solution is partially absorbed by the crotch portion. When lifting the Stride™ underwear from the sheet of liquid absorbent material, it was observed that a substantial amount of the colored liquid solution was absorbed by the sheet, thereby showing that the colored liquid solution had leaked from the Stride™ underwear.

An additional volume of approximately 1 fluid ounce of the prepared color liquid solution was excreted over the outer surface of the inner surface of the crotch portion of the same Stride™ underwear. It was further observed that the liquid-absorbent material had absorbed an additional amount of the colored liquid solution, thereby showing that more colored liquid solution had leaked from the Stride™ underwear.

A volume of approximately 1 fluid ounce of the prepared colored liquid solution was excreted over the outer surface 61 of the water-repellent layer 30 of the undergarment article 2. It was observed that the colored liquid solution permeates through the water-repellent layer 30. When lifting the undergarment article 2 from the sheet of liquid-absorbent material, it was further observed that the liquid-absorbent material did not absorb any of the colored liquid solution, thereby showing that the colored liquid solution was retained by the inner pad 70 and prevented from reaching fabric layer 10 and the liquid-absorbent material.

An additional volume of approximately 1 fluid ounce of the prepared color liquid solution was excreted over the outer surface 61 of the water-repellent layer 30 of the same undergarment article 2. It was further observed that the liquid-absorbent material did not absorb any of the additional volume of the colored liquid solution, thereby showing that the color liquid solution was retained by the inner pad 70 and prevented from reaching the fabric layer 10 and the liquid-absorbent material.

In other tests, further additions of liquid were made and it was observed that the inner pad 70 was efficient to absorb up to about 5 fluid ounces of liquid.

It was found that the Stride™ underwear has a considerably different absorbing layer as compared to the underwear articles of the present disclosure. The Stride™ underwear has an absorbing layer comprising a blue substance similar to a gel that looks like a nonwoven used in disposable products for example disposable products that use a kind of gel to absorb fluids.

### EXPERIMENT 2

According to a second experiment, the heat resistance of the undergarment article 2 was compared with a commercially available liquid-absorbent underwear from Stride™.

In some tests, the undergarment article 2 and the Stride™ underwear were placed in an operating clothes drying machine and submitted to the drying cycles of the machine. These tests showed that the undergarment article 2 described herein is resistant to heat emitted from a clothes drying machine. After the drying cycle, the undergarment article 2 maintained its shape and liquid-absorbent properties. The Stride™ underwear was damaged during the drying cycle.

In some tests, the inner pad 70 of the undergarment article 2 and an absorbent layer of the Stride™ underwear were both exposed to a flame from a conventional lighter placed at about 1 to about 2 cm away for a duration of about 2 to about 3 seconds. The inner pad 70 of the undergarment article 2 resisted the heat from the flame. After being exposed to the flame, the inner pad 70 maintained its shape and liquid-absorbent properties. The absorbent layer of the Stride™ underwear melted when exposed to the flame and was therefore not resistant to the heat from the flame.

### EXPERIMENT 3

In some tests, the elasticity of a crotch portion of the Stride™ underwear was compared with the elasticity of a crotch portion of the undergarment article 2.

It was observed that the crotch portion of the Stride™ underwear was not elastic in neither a longitudinal direction nor a lateral direction. Without being bound to a particular characterization of the Stride™ underwear, it was observed that the inelasticity of the Stride™ underwear may be due to the peripheral stitch of the Stride™ underwear, which joined all of the materials forming the crotch portion of the Stride™ underwear.

Various embodiments of the undergarment article 2 were tested for elasticity. According to embodiments where the water-repellent layer 30 and the crotch portion of the fabric 10 are joined but the inner pad 70 is detached from a transverse stitch or a transverse portion 98 of a continuous stitch 96, it was observed that the water-repellent layer 30 and the crotch portion of the fabric layer 10 are elastic in a longitudinal direction of the crotch portion.

According to embodiments where the water-repellent layer 30 and the crotch portion of the fabric layer 10 are joined but the inner pad 70 is detached from a lateral side of the water-repellent layer 30, it was observed that the water-repellent layer 30 and the crotch portion of the fabric layer 10 are elastic in a longitudinal direction of the crotch portion.

According to embodiments where the water-repellent layer 30 and the crotch portion of the fabric layer 10 are joined but the inner pad 70 is detached from a lateral side of the water-repellent layer 30 and an end of the water-repellent layer 30, and that the water-repellent layer 30, inner pad 70 and fabric layer 10 are joined by stitch 86 positioned remote of a lateral side of the crotch portion, it was observed that the water-repellent layer 30 and crotch portion of the fabric layer 10 are elastic in both a longitudinal direction and lateral direction of the crotch portion.

It was observed that the use of the antimicrobial agent, in combination of the undergarment of the disclosure was quite useful since it allows for extending the life of the undergarment. In fact, while some prior art undergarments are disposable and cannot be used more than once, the use of the antimicrobial agent allows for more efficiently reusing the undergarment of the disclosure. In fact, it helps to eliminate bacteria that would not necessary be killed or eliminated by a common washing and drying process. The antimicrobial agent also provides more comfort to the wearer since the antimicrobial agent at least partially masks undesirable odors. Moreover, the antimicrobial agent maintains the undergarment under clean conditions since being effective for at least partially killing bacteria. By doing so, the customer can thus lower down the costs for each utilization made of such undergarment since the undergarment will have a longer life and can thus be rewashed and reused over an extended period of time.

It was also observed that the use of such an antimicrobial was effective in providing more comfort and by reassuring the wearer since it was effective for masking and/or eliminating undesirable odors. The wearer is thus reassured that body fluid absorbed by the undergarment will not cause or generate odors detectable by other people.

## Claims

1. An undergarment comprising:
a fabric layer (10) for wearing about a pelvic region of a person;
a water-repellent layer (30) coupled to the fabric layer (10), the water-repellent layer (30) and a crotch portion (32) of the fabric layer (10) defining an inner chamber (44), the water-repellent layer (30) comprising polypropylene and being disposed for contacting a genital area of a person wearing the undergarment; and
an inner pad (70) disposed within the inner chamber (44) between the water-repellent layer (30) and the fabric layer (10), the inner pad (70) being reusable and comprising a liquid-absorbent layer (74) and a waterproof layer (78), wherein the liquid-absorbent layer (74) comprises tangled cellulose fibers chosen from bamboo, cotton, viscose and poly-micro; and the waterproof layer (78) comprises polyurethane,
and the inner pad (70) maintaining its shape and liquid-absorbent properties after being submitted to heat, the liquid-absorbent layer (74) contacting the water-repellent layer (30) and being effective for absorbing a volume of liquid of about 88.7 mL (3 fluid ounces) to about 177.4 mL (6 fluid ounces),
the waterproof layer (78) being effective for restricting liquid from reaching the fabric layer (10), and
the water-repellent layer (30) being effective for repelling the liquid and urging the liquid to contact the liquid-absorbent layer (74);
and whereby the undergarment is reusable, renewable and resistant to heat.

2. The undergarment of claim 1, wherein the liquid-absorbent layer (74) absorbs a volume of about 3 fluid ounces (88.7 mL) to about 5 fluid ounces (149.7 mL).

3. The undergarment of claim 1 or 2, wherein the water-repellent layer (30) is liquid-permeable, wherein liquid contacting an exterior surface of the water-repellent layer (30) permeates through the water-repellent layer (30) and reaches an interior surface of the water-repellent layer (30) contacting the liquid-absorbent layer (74), the liquid-absorbent layer (74) absorbing the liquid.

4. The undergarment of any one of claims 1 to 3, wherein the inner pad (70) is cleanable.

5. The undergarment of any one of claims 1 to 4, wherein the heat corresponds to a heat emitted from a clothes drying machine.

6. A kit for reducing discomfort and/or embarrassment caused by incontinency, the kit comprising an undergarment and an antimicrobial agent,
wherein the undergarment comprises:
a fabric layer (10) for wearing about a pelvic region of a person;
a water-repellent layer (30) coupled to the fabric layer (10), the water-repellent layer (30) and a crotch portion (32) of the fabric layer (10) defining an inner chamber (44), the water-repellent layer (30) comprising polypropylene and being disposed for contacting a genital area of a person wearing the undergarment; and
an inner pad (70) disposed within the inner chamber (44) between the water-repellent layer (30) and the fabric layer (10), the inner pad (70) being reusable and comprising a liquid-absorbent layer (74) and a waterproof layer (78), wherein the liquid-absorbent layer (74) comprises tangled cellulose fibers chosen from bamboo, cotton, viscose and poly-micro; and the waterproof layer (78) comprises polyurethane,
and the inner pad (70) maintaining its shape and liquid-absorbent properties after being submitted to heat, the liquid-absorbent layer (74) contacting the water-repellent layer (30) and being effective for absorbing a volume of liquid of about 88.7 mL (3 fluid ounces) to about 177.4 mL (6 fluid ounces),
the waterproof layer (78) being effective for restricting liquid from reaching the fabric layer (10), and
the water-repellent layer (30) being effective for repelling the liquid and urging the liquid to contact the liquid-absorbent layer (74);
and whereby the undergarment is reusable, renewable and resistant to heat;
and wherein the antimicrobial agent comprises a C8-C16 unsaturated fatty acid and/or an essential oil.

7. The kit of claim 6, wherein the antimicrobial agent comprises a fatty acid that is undecylenic acid.

8. The kit of claim 6 or 7, wherein the fatty acid is comprised within microcapsules.

9. A method for reducing discomfort and/or embarrassment caused by incontinency,
the method comprises contacting an undergarment with a C8-C16 unsaturated fatty acid and/or an essential oil and wearing the undergarment, wherein the undergarment comprises:
a fabric layer (10) for wearing about a pelvic region of a person;
a water-repellent layer (30) coupled to the fabric layer (10), the water-repellent layer (30) and a crotch portion (32) of the fabric layer (10) defining an inner chamber (44), the water-repellent layer (30) comprising polypropylene and being disposed for contacting a genital area of a person wearing the undergarment; and
an inner pad (70) disposed within the inner chamber (44) between the water-repellent layer (30) and the fabric layer (10), the inner pad (70) being reusable and comprising a liquid-absorbent layer (74) and a waterproof layer (78), wherein the liquid-absorbent layer (74) comprises tangled cellulose fibers chosen from bamboo, cotton, viscose and poly-micro; and the waterproof layer (78) comprises polyurethane,
and the inner pad (70) maintaining its shape and liquid-absorbent properties after being submitted to heat, the liquid-absorbent layer (74) contacting the water-repellent layer (30) and being effective for absorbing a volume of liquid of about 88.7 mL (3 fluid ounces) to about 177.4 mL (6 fluid ounces),
the waterproof layer (78) being effective for restricting liquid from reaching the fabric layer (10), and
the water-repellent layer (30) being effective for repelling the liquid and urging the liquid to contact the liquid-absorbent layer (74);
and whereby the undergarment is reusable, renewable and resistant to heat.

## Patentansprüche

1. Unterwäsche, die Folgendes umfasst:
eine Stoffschicht (10) zum Tragen um einen Beckenbereich einer Person;
eine wasserabweisende Schicht (30), die mit der Gewebeschicht (10) gekoppelt ist, wobei die wasserabweisende Schicht (30) und ein Schrittabschnitt (32) der Gewebeschicht (10) eine innere Kammer (44) definieren, wobei die wasserabweisende Schicht (30) Polypropylen umfasst und zur Berührung eines Genitalbereichs einer die Unterwäsche tragenden Person angeordnet ist; und
ein Innenkissen (70), das in der Innenkammer (44) zwischen der wasserabweisenden Schicht (30) und der Gewebeschicht (10) angeordnet ist, wobei das Innenkissen (70) wiederverwendbar ist und eine flüssigkeitsabsorbierende Schicht (74) und eine wasserdichte Schicht (78) umfasst, wobei die flüssigkeitsabsorbierende Schicht (74) verwickelte Zellulosefasern umfasst, die unter Bambus, Baumwolle, Viskose und Poly-Mikro ausgewählt sind; und die wasserdichte Schicht (78) Polyurethan umfasst,
und das innere Kissen (70) seine Form und seine flüssigkeitsabsorbierenden Eigenschaften behält, nachdem es der Hitze ausgesetzt wurde, wobei die flüssigkeitsabsorbierende Schicht (74) die wasserabweisende Schicht (30) berührt und zum Absorbieren eines Flüssigkeitsvolumens von etwa 88,7 ml (3 Unzen Flüssigkeit) bis etwa 177,4 ml (6 Unzen Flüssigkeit) wirksam ist,
wobei die wasserdichte Schicht (78) wirksam ist, um zu verhindern, dass die Flüssigkeit die Gewebeschicht (10) erreicht, und
die wasserabweisende Schicht (30) zum Abweisen der Flüssigkeit und zum Drängen der Flüssigkeit zur Berührung mit der flüssigkeitsabsorbierenden Schicht (74) wirksam ist;
und wobei die Unterwäsche wiederverwendbar, erneuerbar und hitzebeständig ist.

2. Unterwäsche nach Anspruch 1, wobei die flüssigkeitsabsorbierende Schicht (74) ein Volumen von etwa 3 Unzen Flüssigkeit (88,7 ml) bis etwa 5 Unzen Flüssigkeit (149,7 ml) absorbiert.

3. Unterwäsche nach Anspruch 1 oder 2, wobei die wasserabweisende Schicht (30) flüssigkeitsdurchlässig ist, wobei Flüssigkeit, die eine Außenfläche der wasserabweisenden Schicht (30) berührt, durch die wasserabweisende Schicht (30) durchtritt und eine Innenfläche der wasserabweisenden Schicht (30) erreicht, die die flüssigkeitsabsorbierende Schicht (74) berührt, wobei die flüssigkeitsabsorbierende Schicht (74) die Flüssigkeit absorbiert.

4. Unterwäsche nach einem der vorstehenden Ansprüche 1 bis 3, wobei das Innenkissen (70) säuberbar ist.

5. Unterwäsche nach einem der vorstehenden Ansprüche 1 bis 4, wobei die Hitze einer von einem Wäschetrockner abgegebenen Hitze entspricht.

6. Kit zur Verringerung von Unbehagen und/oder Verlegenheit, die durch Inkontinenz verursacht werden, wobei der Kit eine Unterwäsche und ein antimikrobielles Mittel umfasst,
wobei die Unterwäsche Folgendes umfasst:
eine Stoffschicht (10) zum Tragen um einen Beckenbereich einer Person;
eine wasserabweisende Schicht (30), die mit der Gewebeschicht (10) gekoppelt ist, wobei die wasserabweisende Schicht (30) und ein Schrittabschnitt (32) der Gewebeschicht (10) eine innere Kammer (44) definieren, wobei die wasserabweisende Schicht (30) Polypropylen umfasst und zur Berührung eines Genitalbereichs einer die Unterwäsche tragenden Person angeordnet ist; und
ein Innenkissen (70), das in der Innenkammer (44) zwischen der wasserabweisenden Schicht (30) und der Gewebeschicht (10) angeordnet ist, wobei das Innenkissen (70) wiederverwendbar ist und eine flüssigkeitsabsorbierende Schicht (74) und eine wasserdichte Schicht (78) umfasst, wobei die flüssigkeitsabsorbierende Schicht (74) verwickelte Zellulosefasern umfasst, die unter Bambus, Baumwolle, Viskose und Poly-Mikro ausgewählt sind; und die wasserdichte Schicht (78) Polyurethan umfasst,
und das innere Kissen (70) seine Form und seine flüssigkeitsabsorbierenden Eigenschaften behält, nachdem es der Hitze ausgesetzt wurde, wobei die flüssigkeitsabsorbierende Schicht (74) die wasserabweisende Schicht (30) berührt und zum Absorbieren eines Flüssigkeitsvolumens von etwa 88,7 ml (3 Unzen Flüssigkeit) bis etwa 177,4 ml (6 Unzen Flüssigkeit) wirksam ist,
wobei die wasserdichte Schicht (78) wirksam ist, um zu verhindern, dass die Flüssigkeit die Gewebeschicht (10) erreicht, und
die wasserabweisende Schicht (30) zum Abweisen der Flüssigkeit und zum Drängen der Flüssigkeit zur Berührung mit der flüssigkeitsabsorbierenden Schicht (74) wirksam ist;
und wobei die Unterwäsche wiederverwendbar, erneuerbar und hitzebeständig ist;
und wobei das antimikrobielle Mittel eine ungesättigte C8-C16-Fettsäure und/oder ein ätherisches Öl umfasst.

7. Kit nach Anspruch 6, wobei das antimikrobielle Mittel eine Fettsäure umfasst, die Undecylensäure ist.

8. Kit nach Anspruch 6 oder 7, wobei die Fettsäure in Mikrokapseln enthalten ist.

9. Verfahren zur Verringerung von Unbehagen und/oder Verlegenheit, die durch Inkontinenz verursacht werden,
wobei das Verfahren das Berühren von Unterwäsche mit einer ungesättigten C8-C16-Fettsäure und/oder einem ätherischen Öl und das Tragen der Unterwäsche umfasst, wobei die Unterwäsche Folgendes umfasst:
eine Stoffschicht (10) zum Tragen um einen Beckenbereich einer Person;
eine wasserabweisende Schicht (30), die mit der Gewebeschicht (10) gekoppelt ist, wobei die wasserabweisende Schicht (30) und ein Schrittabschnitt (32) der Gewebeschicht (10) eine innere Kammer (44) definieren, wobei die wasserabweisende Schicht (30) Polypropylen umfasst und zur Berührung eines Genitalbereichs einer die Unterwäsche tragenden Person angeordnet ist; und
ein Innenkissen (70), das in der Innenkammer (44) zwischen der wasserabweisenden Schicht (30) und der Gewebeschicht (10) angeordnet ist, wobei das Innenkissen (70) wiederverwendbar ist und eine flüssigkeitsabsorbierende Schicht (74) und eine wasserdichte Schicht (78) umfasst, wobei die flüssigkeitsabsorbierende Schicht (74) verwickelte Zellulosefasern umfasst, die unter Bambus, Baumwolle, Viskose und Poly-Mikro ausgewählt sind; und die wasserdichte Schicht (78) Polyurethan umfasst,
und das innere Kissen (70) seine Form und seine flüssigkeitsabsorbierenden Eigenschaften behält, nachdem es der Hitze ausgesetzt wurde, wobei die flüssigkeitsabsorbierende Schicht (74) die wasserabweisende Schicht (30) berührt und zum Absorbieren eines Flüssigkeitsvolumens von etwa 88,7 ml (3 Unzen Flüssigkeit) bis etwa 177,4 ml (6 Unzen Flüssigkeit) wirksam ist,
wobei die wasserdichte Schicht (78) wirksam ist, um zu verhindern, dass die Flüssigkeit die Gewebeschicht (10) erreicht, und
die wasserabweisende Schicht (30) zum Abweisen der Flüssigkeit und zum Drängen der Flüssigkeit zur Berührung mit der flüssigkeitsabsorbierenden Schicht (74) wirksam ist;
und wobei die Unterwäsche wiederverwendbar, erneuerbar und hitzebeständig ist.

## Revendications

1. Sous-vêtement comprenant :
une couche d'étoffe (10) destinée à être portée aux alentours de la région pelvienne d'une personne ;
une couche hydrofuge (30) couplée à la couche d'étoffe (10), la couche hydrofuge (30) et une partie d'entrejambe (32) de la couche d'étoffe (10) définissant une chambre intérieure (44), la couche hydrofuge (30) comprenant du polypropylène étant disposée de manière à venir en contact avec une zone génitale d'une personne portant le sous-vêtement ; et
un coussinet intérieur (70) disposé dans la chambre intérieure (44) entre la couche hydrofuge (30) et la couche d'étoffe (10), le coussinet intérieur (70) étant réutilisable et comprenant une couche absorbant les liquides (74) et une couche étanche à l'eau (78), la couche absorbant les liquides (74) comprenant des fibres de cellulose enchevêtrées choisies parmi le bambou, le coton, la viscose et le poly-micro ; et la couche étanche à l'eau (78) comprenant du polyuréthane,
et le coussinet intérieur (70) conservant sa forme et ses propriétés d'absorption des liquides après avoir été soumis à la chaleur, la couche absorbant les liquides (74) venant au contact de la couche hydrofuge (30) et étant efficace pour absorber un volume de liquide d'environ 88,7 ml (3 onces liquides) à environ 177,4 ml (6 onces liquides),
la couche étanche à l'eau (78) étant efficace pour limiter l'accès d'un liquide à la couche d'étoffe (10), et
la couche hydrofuge (30) étant efficace pour repousser le liquide et pousser le liquide afin qu'il vienne au contact de la couche absorbant les liquides (74) ;
et en conséquence de quoi le sous-vêtement est réutilisable, renouvelable et résistant à la chaleur.

2. Sous-vêtement selon la revendication 1, dans lequel la couche absorbant les liquides (74) absorbe un volume d'environ 88,7 ml (3 onces liquides) à environ 149,7 ml (5 onces liquides).

3. Sous-vêtement selon la revendication 1 ou 2, dans lequel la couche hydrofuge (30) est perméable aux liquides, et dans lequel un liquide venant au contact d'une surface extérieure de la couche hydrofuge (30) traverse par perméation la couche hydrofuge (30) et atteint une surface intérieure de la couche hydrofuge (30) en contact avec la couche absorbant les liquides (74), la couche absorbant les liquides (74) absorbant le liquide.

4. Sous-vêtement selon l'une quelconque des revendications 1 à 3, dans lequel le coussinet intérieur (70) est nettoyable.

5. Sous-vêtement selon l'une quelconque des revendications 1 à 4, dans lequel la chaleur correspond à la chaleur émise par un sèche-linge.

6. Kit pour réduire l'inconfort et/ou l'embarras dus à l'incontinence, le kit comprenant un sous-vêtement et un agent antimicrobien,
dans lequel le sous-vêtement comprend :
une couche d'étoffe (10) destinée à être portée aux alentours de la région pelvienne d'une personne ;
une couche hydrofuge (30) couplée à la couche d'étoffe (10), la couche hydrofuge (30) et une partie d'entrejambe (32) de la couche d'étoffe (10) définissant une chambre intérieure (44), la couche hydrofuge (30) comprenant du polypropylène étant disposée de manière à venir en contact avec une zone génitale d'une personne portant le sous-vêtement ; et
un coussinet intérieur (70) disposé dans la chambre intérieure (44) entre la couche hydrofuge (30) et la couche d'étoffe (10), le coussinet intérieur (70) étant réutilisable et comprenant une couche absorbant les liquides (74) et une couche étanche à l'eau (78), la couche absorbant les liquides (74) comprenant des fibres de cellulose enchevêtrées choisies parmi le bambou, le coton, la viscose et le poly-micro ; et la couche étanche à l'eau (78) comprenant du polyuréthane,
et le coussinet intérieur (70) conservant sa forme et ses propriétés d'absorption des liquides après avoir été soumis à la chaleur, la couche absorbant les liquides (74) venant au contact de la couche hydrofuge (30) et étant efficace pour absorber un volume de liquide d'environ 88,7 ml (3 onces liquides) à environ 177,4 ml (6 onces liquides),
la couche étanche à l'eau (78) étant efficace pour limiter l'accès d'un liquide à la couche d'étoffe (10), et
la couche hydrofuge (30) étant efficace pour repousser le liquide et pousser le liquide afin qu'il vienne au contact de la couche absorbant les liquides (74) ;
et en conséquence de quoi le sous-vêtement est réutilisable, renouvelable et résistant à la chaleur ;
et dans lequel l'agent antimicrobien comprend un acide gras insaturé en C₈ à C₁₆ et/ou une huile essentielle.

7. Kit selon la revendication 6, dans lequel l'agent antimicrobien comprend un acide gras qui est l'acide undécylénique.

8. Kit selon la revendication 6 ou 7, dans lequel l'acide gras est compris à l'intérieur de microcapsules.

9. Procédé pour réduire l'inconfort et/ou l'embarras dus à l'incontinence,
le procédé comprend la mise en contact d'un sous-vêtement avec un acide gras insaturé en C₈ à C₁₆ et/ou une huile essentielle et le fait de porter le sous-vêtement, dans lequel le sous-vêtement comprend :
une couche d'étoffe (10) destinée à être portée aux alentours de la région pelvienne d'une personne ;
une couche hydrofuge (30) couplée à la couche d'étoffe (10), la couche hydrofuge (30) et une partie d'entrejambe (32) de la couche d'étoffe (10) définissant une chambre intérieure (44), la couche hydrofuge (30) comprenant du polypropylène étant disposée de manière à venir en contact avec une zone génitale d'une personne portant le sous-vêtement ; et
un coussinet intérieur (70) disposé dans la chambre intérieure (44) entre la couche hydrofuge (30) et la couche d'étoffe (10), le coussinet intérieur (70) étant réutilisable et comprenant une couche absorbant les liquides (74) et une couche étanche à l'eau (78), la couche absorbant les liquides (74) comprenant des fibres de cellulose enchevêtrées choisies parmi le bambou, le coton, la viscose et le poly-micro ; et la couche étanche à l'eau (78) comprenant du polyuréthane,
et le coussinet intérieur (70) conservant sa forme et ses propriétés d'absorption des liquides après avoir été soumis à la chaleur, la couche absorbant les liquides (74) venant au contact de la couche hydrofuge (30) et étant efficace pour absorber un volume de liquide d'environ 88,7 ml (3 onces liquides) à environ 177,4 ml (6 onces liquides),
la couche étanche à l'eau (78) étant efficace pour limiter l'accès d'un liquide à la couche d'étoffe (10), et
la couche hydrofuge (30) étant efficace pour repousser le liquide et pousser le liquide afin qu'il vienne au contact de la couche absorbant les liquides (74) ;
et en conséquence de quoi le sous-vêtement est réutilisable, renouvelable et résistant à la chaleur.
